(19) Europäisches Patentamt
European Patent Office
Office européen des brevets

(11) Veröffentlichungsnummer : **0 034 738**
**B1**

(12) # EUROPÄISCHE PATENTSCHRIFT

(45) Veröffentlichungstag der Patentschrift : **15.05.85**

(21) Anmeldenummer : **81100854.9**

(22) Anmeldetag : **06.02.81**

(51) Int. Cl.[4] : **C 07 C 45/40**, C 07 C 27/16, C 07 C 51/34, C 07 C 49/433, C 07 B 33/00, C 07 B 35/04

(54) **Verfahren zur kontinuierlichen Ozonolyse von Beta-Pinenen.**

(30) Priorität : **14.02.80 DE 3005514**

(43) Veröffentlichungstag der Anmeldung : **02.09.81 Patentblatt 81/35**

(45) Bekanntmachung des Hinweises auf die Patenterteilung : **15.05.85 Patentblatt 85/20**

(84) Benannte Vertragsstaaten : **CH DE LI**

(56) Entgegenhaltungen :
**DE-A- 2 713 863**
**DE-A- 2 754 366**
**ZEITSCHRIFT FÜR ANGEWANDTE CHEMIE, Band 42, 1929, Berlin H. SCHMIDT "Ozonisation des Nopinens und Sabinens", Seiten 126 bis 127**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Witthaus, Martin, Dr.**
**Burgmüller Strasse 1**
**D-4000 Düsseldorf 1 (DE)**
Erfinder : **Carduck, Franz-Josef, Dr.**
**Landstrasse 18**
**D-5657 Haan (DE)**
Erfinder : **Maymudar, Suresh, Dr.**
**Mozartstrasse 27**
**D-5657 Haan (DE)**

**EP 0 034 738 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Ozonolyse olefinischer Verbindungen erfolgt in der Regel in den zwei Stufen der Anlagerung von Ozon an die Doppelbindung in Gegenwart inerter oder reaktiver Lösungsmittel und der nachfolgenden Spaltung der Zwischenprodukte aus Stufe 1. Diese Spaltung erfolgt üblicherweise hydrolytisch, oxydativ oder reduktiv. Eine parallel ablaufende thermische Spaltung führt zu unerwünschten Nebenreaktionen und muß daher möglichst unterdrückt werden. Die hierfür am häufigsten angewandte Methode ist die Ozonisierung bei tiefen Temperaturen (≤ 0 °C) mit anschließender Spaltung bei höheren Temperatur (50° bis 130 °C). Durch die Mitverwendung reaktiver Lösungsmittel, insbesondere Alkohole oder Carbonsäuren können die Ozonisierungsprodukte aus Stufe 1 unter intermediärer Bildung von Alkoxy- oder Acyloxyhydroperoxiden stabilisiert werden.

Eine besonders elegante Ozonolyse-Technik, insbesondere für solche Olefine, deren Ozonisierungsprodukte hydrolytisch gespalten werden sollen, besteht in der Mitverwendung von Wasser bereits in der Ozonisierungsstufe. Hier kommt dem zugesetzten Wasser, das im Olefin dispergiert werden muß, eine doppelte Funktion zu : Abführung der sehr hohen Reaktionswärme durch in situ Verdunstungskühlung bei Temperaturen um 30 bis 50 °C sowie Unterdrückung der bei diesen Temperaturen bereits einsetzenden Nebenreaktionen durch die als gewünschte Konkurrenzreaktion ablaufende hydrolytische Spaltung.

In der DE-OS 27 54 366 ist ein Verfahren und eine Vorrichtung zum kontinuierlichen Ozonisieren von höhermolekularen Olefinen und ungesättigten Fettsäuren beschrieben, bei dem man eine Emulsion von Wasser in der Lösung des olefinischen Ausgangsmaterials in einem reaktiven organischen Lösungsmittel mit einem Ozon enthaltenden Gasstrom behandelt und das Reaktionsprodukt der hydrolytischen Spaltung unterwirft. Der flüssige und der gasförmige Reaktant werden dabei im Gleichstrom geführt und die Reaktionstemperatur wird so gesteuert, daß sie maximal 50 °C beträgt. Der Ozonisierungsschritt des Verfahrens kann in einem mit statischen Mischelementen beschickten Kolonnenreaktor durchgeführt werden. Die in der DE-OS 27 54 366 beschriebene Verfahrensweise ist eine Abwandlung und Weiterentwicklung eines in der DE-OS 27 13 863 beschriebenen Verfahrens zur Ozonisierung von Olefinen und ungesättigten Fettsäuren, mit dem es in den wesentlichen Merkmalen weitgehend übereinstimmt.

In der Literatur finden sich auch Arbeiten zur Ozonolyse von Terpen-Olefinen und insbesondere zur Gewinnung von Nopinon aus β-Pinen gemäß

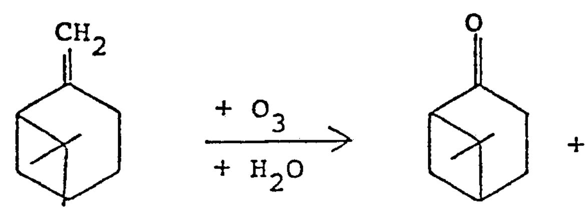


(Formaldehyd
(Ameisensäure
(Perameisensäure

β-Pinen Nopinon

Nach den Literaturangaben, z. B. G. Brus und G. Pyresblauques ; Compt. rend. *187*(1928) 984 oder H. Schmidt ; Angew. Chem. *42* (1929) 126, C. Harries et al. ; Ber. Dtsch. Chem. Ges. *41* (1908), 38 *42* (1909) 879, Chem. Ztrbl. 1916, II. 994, wird die Ozonisierung von β-Pinen üblicherweise in Methanol (1 : 5 bis 1 : 10) bei Temperaturen von − 10 °C bis 0 °C mit anschließender Wasserdampfspaltung durchgeführt. Synthesen mit chlorierten Kohlenwasserstoffen als Lösungsmittel bei Temperaturen bis + 10 °C wurden ebenfalls beschrieben. Alle diese Verfahren sind Laborsynthesen. Sie eignen sich nur bedingt für die technische Produktion, und zwar insbesondere aus den folgenden Gründen :

1. Bei Ozonkonzentrationen von nur 1 bis 3 Vol.-% werden große Menge Lösungsmittel mit der Abluft ausgetragen und erfordern wegen Explosionsgefahr umfangreiche Abgasreinigungsanlagen und Sicherheitsmaßnahmen.
2. Die Abführung der hohen Reaktionsthalpie von ca. 100 kcal/Mol ist bei tiefen Temperaturen aufwendig (Solekühlung).
3. Eine diskontinuierliche Ozonisierung ist für die technische Produktion unpraktisch.

Vergleichbare Schwierigkeiten sind für die Ozonisierung von hochreaktiven Monoolefinen gegeben, die ihre Doppelbindung in « endständiger » Position — d. h. in α-, β- und/oder γ-Stellung — aufweisen. Die Ozonolyse von Monoolefinen dieser Art hat praktisches technisches Interesse beispielsweise für die Gewinnung von Carbonsäuren aus Olefinen, ist jedoch bisher mit so schwerwiegenden technischen Nachteilen behaftet, daß eine praktische Verwertung dieser Möglichkeit nicht stattfindet. Die besonderen Schwierigkeiten, die hier und im Fall der vorher erörterten Terpenozonolyse vor allem zu bewältigen sind, liegen in der Abspaltung vergleichsweise hochflüchtiger, zur Selbstentzündung neigender Spaltprodukte. Bei α-Olefinen werden beispielsweise entsprechende Bruchstücke mit nur einem Kohlenstoffatom gebildet, die eine sichere Bewältigung des Verfahrens im technischen Maßstab beträchtlich erschweren.

Aufgabe der Erfindung ist die Schaffung eines Verfahrens, mit dem in kontinuierlichen Verfahren großtechnisch β-Pinen gefahrlos bei guten Ausbeuten ozonisiert werden kann und die dabei entstehenden Zwischenprodukte der anschließenden Spaltung unterworfen werden können. Die Erfindung hat sich insbesondere die Aufgabe gestellt, ein großtechnisch brauchbares kontinuierliches Verfahren zur Gewinnung von Nopinon aus β-Pinen durch Ozonolyse und anschließende hydrolytische Spaltung des Zwischenproduktes zu schaffen.

Gegenstand der Erfindung ist ein Verfahren zur kontinuierlichen Ozonolyse von β-Pinen durch Behandeln einer Emulsion von Wasser in der Lösung des olefinischen Ausgangsmaterials in einem reaktiven organischen Lösungsmittel mit einem 0,5 bis 1,5 Gew.-% Ozon enthaltenden Gasstrom bei Gleichstromführung von flüssigem und gasförmigem Reaktanten, wobei die maximale Reaktionstemperatur auf Werte von höchstens 50 °C gesteuert wird, und Aufarbeitung des Reaktionsproduktes durch hydrolytische Spaltung, das dadurch gekennzeichnet ist, daß man unter Vermeidung der Ausbildung von Flüssigkeitsnestern im Reaktor den flüssigen Reaktanten als turbulent strömenden Film großer Oberfläche der Einwirkung des gasförmigen Reaktanten aussetzt, dabei den gasförmigen Reaktanten mit Leerrohr-Fließgeschwindigkeiten von wenigstens 2 m/sec und Verweilzeiten von weniger als 2 sec im Reaktor in hochturbulenter Zick-Zack-Strömung auf bzw. über den Flüssigkeitsfilm führt und im Abgasstrom auftretende Reaktionsnebel mittels eines auf der Anströmseite mit einer Flüssigkeit beaufschlagten Tiefenfilters bricht.

Bei der Erarbeitung des erfindungsgemäßen Verfahrens hat sich überraschenderweise gezeigt, daß die Umsetzung an sich bekannter Verfahrensprinzipien, wie sie beispielsweise in der DE-OS-27 13 863 beschrieben sind, auf die Umwandlung von β-Pinen zu Nopinon beträchtliche Schwierigkeiten für ein großtechnisch durchführbares kontinuierliches Verfahren macht. Diese Schwierigkeiten leiten sich dabei aus mehreren Quellen ab.

β-Pinen kann mit seiner $=CH_2$-Gruppe in der Seitenkette als α-Olefin verstanden werden. Bei der Ozonolyse bilden sich unter Spaltung der Doppelbindung leicht flüchtige organische Peroxide, die — insbesondere in kondensiertem Zustand — im Reaktionsmedium (ozonhaltige Luftstrom) selbst-entzündlich sind. Es zeigt sich weiterhin, daß die im erfindungsgemäßen Verfahren als flüssige Reaktionspartner eingesetzte Wasser-in-Öl-Emulsion sehr viel leichter zum Brechen und zur Entmischung neigt als vergleichbare Emulsionen der Ozonolyse von Ölsäure gemäß DE-OS 27 13 863. Im Abgas der Ozonisierungsstufe tritt mit steigender Reaktionstemperatur in zunehmendem Maße die Bildung selbstentzündlicher Nebel auf, so daß eine Abgasreinigung zwingend erforderlich werden kann. Eine übliche auch mehrfache Gaswäsche mit 4 %-iger Natriumsulfitlösung im Venturiwäscher mit nachgeschalteter Füllkörperkolonne beseitigt die Nebel nur in unbefriedigendem Maße. Herkömmliche Aerosolabscheider, die nach dem Diffusionsprinzip mit Gasgeschwindigkeiten $< 0,05$ m/sec. arbeiten, kommen wegen der Selbstentzündlichkeit des Kondensats nicht in Betracht. Schließlich ist das Druckpotential des Verfahrens aufgrund der Konstruktion üblicher Ozoneure mit 1,6 bis 1,8 bar begrenzt, wenn nicht zusätzliche Schwierigkeiten in das Verfahren eingebaut werden sollen.

Trotz dieser Schwierigkeiten gelingt im erfindungsgemäßen Verfahren die gewünschte Bildung von Nopinon aus β-Pinen im kontinuierlichen Verfahren mit guten Ausbeuten.

Wichtig für die Lehre der Erfindung ist zunächst die Art und Weise der Zusammenführung der Reaktionspartner im Ozonisierungsreaktor. Der flüssige und gasförmige Reaktionspartner werden im Gleichstrom durch den Reaktionsraum geführt. Hierbei sind jedoch besondere Bedingungen einzuhalten.

Für die Phase des flüssigen Reaktionspartners ist entscheidend, daß die Bildung von Flüssigkeitsnestern vermieden wird, wie sie üblicherweise auch in Austauscherkolonnen mit Einbauten oder Füllkörpern auftreten und toleriert werden können. Üblicherweise ist eine solche Bildung von Flüssigkeitsnestern in mehr oder weniger großer Zahl unbedenklich, weil Gefahren für das Verfahren von ihnen nicht ausgehen und die Verweilzeit eines individuellen Flüssigkeitsteilchens im Nest nicht lang ist. Erfindungsgemäß ist jedoch darauf zu achten, daß der flüssige Reaktionspartner über den gesamten Reaktionsraum ohne Ausbildung von stagnierenden Flüssigkeitszusammenballungen als turbulent strömender dünner Film mit großer Oberfläche dem gasförmigen Reaktionspartner ausgesetzt wird und bleibt. Überraschenderweise sind in dieser Form der Reaktionsführung die Gefahren der Selbstentzündung gebannt, obwohl an der chemischen Beschaffenheit des Reaktionsmediums selbst keine Veränderung vorgenommen werden.

Die Führung der Flüssigphase in turbulenter Filmströmung hat eine mehrfache Wirkung : Zunächst ist die ständige Reemulgierung des Wassers in der organischen Flüssigphase und damit dessen feinste Verteilung im flüssigen Reaktionsmedium gewährleistet. Hierdurch wird die zuverlässige Temperaturkontrolle in allen Bereichen des flüssigen Reaktionspartners durch in-situ-Verdampfung des Wassers sichergestellt. Zum anderen ist damit gewährleistet, daß die Diffusion von Ozon aus der Gasphase in die Flüssigphase als geschwindigkeitsbestimmender Schritt der erfindungsgemäßen Umsetzung so stark beschleunigt wird, daß damit die hohe Reaktionsgeschwindigkeit des erfindungsgemäßen Ausgangsmaterials bei seiner Umsetzung mit Ozon im Verfahren ausgenutzt werden kann. Schließlich wird die Bildung von gefährlichen Ablagerungen vermieden.

Diese hohe Reaktionsgeschwindigkeit ermöglicht die erfindungsgemäß bestimmte Führung des gasförmigen Reaktionspartners. Charakteristisch sind hierfür extrem hohe Fließgeschwindigkeiten der Gasphase bei extrem kurzen Verweilzeiten im Reaktor.

Die Fließgeschwindigkeiten — bestimmt als Leerrohr-Fließgeschwindigkeit — betragen wenigstens 2 m/sec. Bevorzugt wird bei höheren Fließgeschwindigkeiten gearbeitet, insbesondere solchen von wenigstens 5 m/sec. Die obere Grenze der Fließgeschwindigkeit liegt üblicherweise bei etwa 15 m/sec., wobei Fließgeschwindigkeiten im Bereich von 7 bis 12 m/sec. besondere Bedeutung besitzen können. Alle diese Zahlenangaben beziehen sich dabei auf die Leerrohr-Fließgeschwindigkeit. Da tatsächlich nicht mit einem leeren Rohr, sondern mit einem mit Einbauten versehenen Reaktor gearbeitet wird und der Gasstrom durch eine Vielzahl von Kanälen geführt wird, liegen die effektiven Gasgeschwindigkeiten beträchtlich über den hier genannten Zahlenwerten.

Die Verweilzeit des Gasstroms ist bei diesen hohen Fließgeschwindigkeiten im Reaktor nur kurz. Sie liegt bevorzugt unter 1 sec. und vorzugsweise unter 0,7 sec. Besonders bevorzugt kann es sein, mit Verweilzeiten des gasförmigen Reaktionsteilnehmers im Reaktor unter 0,5 sec. zu arbeiten.

Aufgrund der hohen Fließgeschwindigkeiten und der im folgenden noch im einzelnen geschilderten Ausgestaltung des Reaktorinnenraumes wird der gasförmige Reaktionspartner in hochturbulenter Zick-Zack-Strömung durch den Reaktorraum gedrückt und trifft dabei in einer unendlichen Zahl von Wiederholungen auf den in dünner Schicht vorliegenden bzw. durch den Reaktor wandernden flüssigen Reaktionsteilnehmer. Dieses forcierte Zusammenführen von flüssigem und gasförmigen Reaktionspartner geforderte Turbulenz der vorliegenden Filmschicht. Die hohen Fließgeschwindigkeiten des gasförmigen Reaktionspartners wirken schließlich der Bildung von Flüssigkeitsnestern durch einfaches Ausblasen entgegen, so daß in synergistischem Zusammenwirken nicht nur die Umsetzung der beiden Reaktionspartner ermöglicht, sondern gleichzeitig auch das Aufrechterhalten der geforderten bestimmten Beschaffenheit der Reaktionszone gewährleistet ist.

Das als Ausgangsmaterial eingesetzte Olefin und Ozon werden in etwa äquivalentem Verhältnis in die Reaktion eingebracht. Es kann dabei zweckmäßig sein, durch einen Vorversuch zunächst zu ermitteln, wie hoch der zur quantitativen Umsetzung des eingesetzten Olefins erforderliche Ozonbedarf ist. Bei der Umsetzung von β-Pinen unter den erfindungsgemäßen Verfahrensbedingungen hat sich herausgestellt, daß der Ozonbedarf zur quantitativen Umsetzung des β-Pinens ca. 80 bis 85 % der Theorie betragen kann. Überschuß-Ozon wird infolge der sehr kurzen Verweilzeit des Gases im Reaktor nur in geringem Maße durch die erheblich langsameren Nebenreaktionen verbraucht und erscheint daher fast quantitativ im Abgas. Ein leichter Ozonüberschuß oder -unterschuß (z. B. bis zu 5 % — bezogen auf die benötigte Ozonmenge) kann von Vorteil sein.

Im erfindungsgemäßen Verfahren gelingt die Umsetzung der Reaktanten trotz der extrem kurzen Verweilzeit des gasförmigen Reaktionspartners im Reaktionsraum mit Umsatzausbeuten von wenigstens 97 %, vorzugsweise von 98 und mehr Prozent.

Die Reaktion findet in einem mit geometrisch regelmäßigen Einbauten bestückten Reaktor statt. Wesentliche Kriterien für die Auswahl der Packung sind : Geringer Druckverlust, Vermeidung von Flüssigkeits-Toträumen, Vermeidung von by-pass-Strömungen der Luft, große Oberfläche, Selbstreinigung.

Die geforderten Bedingungen werden beispielsweise durch handelsübliche statische Mischelemente erfüllt. Um eine hinreichend große Oberfläche zu erhalten, muß eine große Zahl derartiger Mischelemente in den Reaktor eingebaut werden. Es wurde gefunden, daß auch mit einfacheren Einbauten vergleichbar gute Verfahrensergebnisse erzielt werden können. Geeignet sind beispielsweise Einbaukörper aus gewellten und vielfach abgeknickten, sich kreuzenden Streckmetallbändern wie sie als Füllungen für Rektifikations- und Absorbtionskolonnen mit besonderen Vorteilen, insbesondere im mittleren Vakuumbereich bis zu Normaldruck und darüber bekannt sind. In dem hier vorliegenden System sich kreuzender Kanäle ist die hochturbulente Zick-Zack-Strömung des gasförmigen Reaktanten gewährleistet. Als Reaktoreinbauten können beispielsweise die im Prospekt VT 2002/d (Mai 1977) der Firma Gebr. Sulzer AG, Winterthur, Schweiz, dargestellten Formkörper (« Mellapak » Typ 250 Y) Verwendung finden. Bezüglich weiterer Einzelheiten wird verwiesen auf Chem. Ind. Tech. 51 (1979), Nr. 12, 1 151-1 158, insbesondere die Angaben zu « Füllkörperpackungen » a. a. O., Seiten 1 154-1 155, linke Spalte, oben.

Die Gesamtoberfläche im Reaktorinnenraum, auf der sich der flüssige Reaktionsteilnehmer in dünner Schicht bewegt, sollte wenigstens etwa 50 $m^2/m^3$ betragen. Bevorzugt sind wesentlich höhere Werte. So sind Oberflächen von wenigstens 100 $m^2/m^3$ und insbesondere solche von wenigstens 100 $m^2/m^3$ bevorzugt. Besonders geeignet sind beispielsweise Oberflächen von wenigstens 200 $m^2/m^3$, wobei die zur Verfügung gestellte Fläche nach oben hin lediglich dadurch begrenzt wird, daß die hohen Fließgeschwindigkeiten des gasförmigen Reaktionspartners bei dem in der Regel doch nur beschränkten Druckpotential sichergestellt sein müssen.

Die erfindungsgemäße Umsetzung kann in einem einstufigen Reaktor mit vorzugsweise abwärts strömendem Reaktionsgemisch durchgeführt werden. Trotz einstufiger Arbeitsweise und der Notwendigkeit einer optimalen Umsetzung sind Füllkörperreaktoren nur beschränkter Länge von beispielsweise 2 bis 5 m geeignet. Gewünschtenfalls kann im mehrstufigen Verfahren gearbeitet werden. Je nach Temperaturführung, die im folgenden noch im einzelnen erörtert wird, kann eine Zusatzkühlung von außen vorgesehen sein. Hier empfiehlt sich in der Regel das Arbeiten mit einem Rohrbündel-Reaktor.

In der für die Praxis üblicherweise bevorzugten Ausführungsform wird auf eine Außenkühlung des Reaktionsraums verzichtet. Die Abführung der hohen Reaktionswärme erfolgt durch die Verdunstung

und damit die in-situ-Kühlung von Anteilen des Wassers aus dem flüssigen Reaktionspartner. Im übrigen wird die Reaktionstemperatur durch den Ozongehalt des gasförmigen Reaktionstemperatur durch den Ozongehalt des gasförmigen Reaktionspartners gesteuert.

In dieser bevorzugten Verdunstungskühlung mittels des emulgierten Wassers stellt sich im Reaktionsraum ein Gleichgewichtstemperaturprofil ein, das eindeutig definiert und über die Konzentration des Ozons im eingesetzten $O_3$/Luftgemisch steuerbar ist. Bei hohen Ozonkonzentrationen stellen sich höhere Reaktionstemperaturen ein und umgekehrt.

Bei der Abführung der Reaktionswärme nur durch Verdunstungskühlung stellen sich im Gleichgewicht Maximaltemperaturen von 35 bis 45 °C ein. Wird beispielsweise Ozon in einer Konzentration von 10 g/Nm$^3$ Luft eingesetzt und nahezu quantitativ umgesetzt, dann kann ein Temperaturgradient zwischen beispielsweise 20 °C (± 0,5 °C) am Reaktoreingang und 38 °C (± 2 °C) am Reaktorausgang eingestellt werden. Mit 15 g/Nm$^3$ stellt sich bei gleichem Luftdurchsatz und ebenfalls quantitativem Umsatz ein Temperaturprofil von 20 °C (Eintritt) bis 43 °C (Austritt) ein.

Anstelle des Arbeitens mit einem solchen Temperaturprofil im Bereich von 20° bis 50 °C ist es auch möglich, die Maximaltemperaturen auf unter 20° bis 25 °C abzusenken. Hierzu ist es allerdings notwendig, den Reaktionsraum von außen, beispielsweise durch Sole, zu kühlen. Im übrigen gilt auch hier das Prinzip der — zusätzlichen — Temperatursteuerung durch die Ozonkonzentration im gasförmigen Reaktionspartner. Möglich ist es in dieser Ausführungsform der Erfindung beispielsweise im Temperaturbereich von 10° bis 20 °C zu arbeiten.

Diese Senkung der Verfahrenstemperatur bringt zwar eine gewisse Erschwerung mit sich, andererseits wird die Produktausbeute günstig beeinflußt.

Temperaturabhängig ist darüberhinaus auch eine weitere Schwierigkeit des erfindungsgemäßen Verfahrens : Je höher die Reaktionstemperatur ist, umso stärker tritt eine Bildung von Nebeln im Abgas auf. Die Nebeltröpfchen enthalten leichtflüchtige organische Peroxide, die in kondensiertem Zustand selbstentzündlich sind. Man muß also nicht nur darauf achten, daß die Nebelbildung insgesamt beschränkt bleibt, insbesondere ist eine Abgasreinigung erforderlich, sofern solche Nebel in nicht lediglich unbeträchtlicher Menge entstanden sind.

Wegen der zuvor geschilderten Schwierigkeiten bei dieser Nebelabscheidung, insbesondere auch wegen der Selbstentzündlichkeit des Kondensats, wurde für dieses spezielle Abgasreinigungssystem eine Lösung entwickelt, in dem das nebelhaltige Abgas vorzugsweise im Abstrom durch eine Tiefenfilterplatte abgeleitet wird, wobei die Filterplatte auf der Anströmseite mit einer Flüssigkeit besprüht wird. Als Flüssigphase kann hier beispielsweise das flüssige Reaktionsgemisch nach Verlassen des Ozonisierungsreaktors oder Anteile hiervon eingesetzt werden. In einer bevorzugten Ausführungsform der Erfindung wird das gesamte flüssige Reaktionsgemisch aus der Ozonisierungsstufe auf die Anströmseite des Tiefenfilters geführt und mit dem Gasstrom durch den Tiefenfilter hindurchgedrückt. Hierbei werden die aus dem Nebel abgeschiedenen peroxidhaltigen Aerosoltröpfchen quantitativ im Reaktionsgemisch gelöst und durch sofortige Überführung in die hydrolytische Spaltungsstufe unschädlich gemacht. Alternativ kann die Nebelabscheidung — nach Abtrennung des flüssigen Reaktionsgemisches — in der Abgasleitung erfolgen, wobei die wieserum horizontal eingebaute Tiefenfilterplatte von oben mit Wasser besprüht wird. Die Aerosolteilchen treten dabei an der Unterseite der Filterplatte als ungefährlilche Öl-in-Wasser-Emulsion aus, die beispielsweise mit dem Abgasstrom in einen nachgeschalteten Natriumsulfitwäscher überführt werden kann, der für die Beseitigung von Überschußozon ohnehin zweckmäßig oder erforderlich ist. Hier werden die aus dem Nebel abgeschiedenen peroxidhaltigen Aerosoltröpfchen reduziert und damit unschädlich gemacht.

Bevorzugt wird erfindungsgemäß weiterhin die Tiefenfilterplatte dicht im Anschluß an den Austritt des Ozonisierungsreaktors vorgesehen, wobei der Querschnitt der Tiefenfilterplatte dem Reaktorquerschnitt etwa entsprechen kann, so daß das gesamte die Ozonisierungsstufe verlassende Reaktionsprodukt — gasförmiger und flüssiger Bestandteil — durch diese Tiefenfilterplatte geleitet wird.

Auch wegen dieser möglichen Nebelbildung ist im erfindungsgemäßen Verfahren die Einschränkung des Ozongehalts im gasförmigen Reaktionspartner wünschenswert oder gar erforderlich. Allgemein gilt, daß die Ozonkonzentrationen im Luftstrom im Bereich von 0,5 bis 1,5 Gew.-% und vorzugsweise im Bereich von 1 bis 1,3 Gew.-% liegen können. Als besonders zweckmäßig hat sich das Arbeiten mit 7 bis 15 g Ozon/Nm$^3$ des gasförmigen Reaktionspartners erwiesen.

Als Tiefenfilter sind entsprechende Elemente geeignet, die mit den erfindungsgemäß eingesetzten Gasgeschwindigkeiten von beispielsweise 3 bis 15 m/sec. angeströmt werden können. Als Tiefenfilter eignen sich beispielsweise Keramikplatten, Sintermetallplatten und vorzugsweise Faserschichten aus Glaswolle, Steinwolle, Kunststoff oder Metall. Um die Ansammlung der selbstentzündlichen Aerosoltröpfchen im Filterelement zu vermeiden, werden die Platten zweckmäßigerweise horizontal am Ende des Reaktors eingebaut und von oben nach unten gleichzeitig mit Abgas und flüssigem Reaktionsgemisch durchströmt. Der Strömungswiderstand der Filterplatten ist so gering wie möglich zu halten, andererseits muß natürlich die Nebelabscheidung gewährleistet sein. Es hat sich gezeigt, daß beispielsweise mit zwei übereinandergelegten Metallfaserplatten von je ca. 0,6 mm Stärke (40 µ Filterfeinheit) eine quantitative Nebelabscheidung bei nur geringem Druckverlust erzeilt werden kann. Durch das Auswaschen des Aerosols mit dem Reaktionsgemisch wird nicht nur die Abgasreinigung und die Überführung der selbstentzündlichen Tröpfchen in eine ungefährliche Wasser/Öl-Emulsion erreicht, es wird insbesondere

auch die Rückgewinnung von Reaktionsprodukt aus dem Abgas erleichtert.

In der jetzt folgenden Reaktionsstufe erfolgt die hydrolytische Spaltung. Das Ozonisierungsprodukt wird als Wasser/Öl-Emulsion unmittelbar nach Austritt aus dem Ozonisierungsreaktor in einen Spaltreaktor gepumpt, der zweckmäßigerweise kontinuierlich betrieben und dabei mehrstufig ausgelegt sein kann. Geeignet sind beispielsweise zweistufige Spaltreaktoren, deren beide Stufen durch Einleiten von Niederdruckdampf bei einer Temperatur von ca. 100° bis 105 °C gefahren werden. Der Reaktor kann z. B. aus zwei hintereinander geschalteten mit Dampf gerührten Behältern bestehen oder aus einer von unten nach oben mit Dampf/Ozonid im Gleichstrom durchflossenen Säule, die mittels Siebboden in zwei Kammern unterteilt ist. Überschüssiger Dampf wird in einem Rückflußkühler niedergeschlagen. Für eine quantitative Spaltung ist üblicherweise eine Verweilzeit von ca. 40 bis 80 Min. ausreichend.

Zur Beschaffenheit und Zusammensetzung des bevorzugten flüssigen Reaktionspartners gelten die folgenden Angaben : Als reaktive Lösungsmittel eignen sich prinzipiell schwerflüchtige Alkohole und Carbonsäuren, deren relative Flüchtigkeit im Verhältnis zum gewünschten Verfahrensprodukt so abgestimmt ist, daß eine einfache destillative Aufarbeitung der Spaltprodukte möglich ist. Andererseits sollte das Lösungsmittel bei den eingesetzten niedrigsten Verfahrenstemperaturen noch nicht erstarren.

Zur Nopinonsynthese haben sich besonders Caprylsäure und Perlargonsäure als reaktive Lösungsmittel bewährt. Insgesamt geben hier Säuren bessere Ausbeuten als Alkohole, wie beispielsweise Decanol, wenn deren Anwendung auch nicht ausgeschlossen ist.

Auf 1 Mol-Teil des eingesetzten der Regel 0,5 bis 5 Mol-Teile des reaktiven Lösungsmittels, insbesondere 0,7 bis 3 Mol-Teile des reaktiven Lösungsmittels, verwendet. Wasser wird in diesem flüssigen Reaktionspartner in Mengen von 10 bis 50 Mol-Teilen, vorzugsweise 10 bis 30 Mol-Teilen emulgiert. Die bereits im Reaktor durch hydrolytische Spaltung der Esterhydroperoxide wieder frei werdende Säure steht erneut zur stabilisierenden Veresterung der primären Ozonisierungsprodukte zur Verfügung. Hierdurch ist es möglich, mit einem vergleichsweise geringen Lösungsmittelbedarf zu arbeiten. Zu berücksichtigen ist lediglich, daß die Fließfähigkeit des im Verlauf der Reaktion viskoser werdenden flüssigen Reaktanten erhalten bleibt.

Im Rahmen des hier geschilderten Verfahrens zur Oxydation von β-Pinen, hat sich eine weitere Modifikation des Verfahrenskreislaufs als vorteilhaft erwiesen : Im Rahmen der Erfindung ist es möglich, nopinonhaltiges Spaltprodukt, das aus der Stufe der hydrolytischen Spaltung abgezogen wird, in die Ozonisierungsstufe zurückzuführen. Es ist dabei möglich, beispielsweise bis zu 50 % des Produktes aus der hydrolytischen Spaltung in die Ozonisierung zurückzuführen. Es wird hier also Nopinon, reaktives Lösungsmittel, insbesondere reaktive Carbonsäure und Wasser enthaltendes Spaltprodukt, vorzugsweise oben in die Ozonisierungsstufe zurückgeführt. Hierdurch wird die Anreicherung des Produktstromes an Nopinon erhöht. So kann es beispielsweise möglich sein, den Produktstrom auf diese Weise bis zu einem Gehalt von 40 bis 45 % Nopinon anzureichern während ohne Rückführung im Produktstrom beispielsweise nur ca. 25 % des erwünschten Reaktionsproduktes vorliegen. Eine Schädigung des Nopinons durch diese Kreislaufführung des hydrolytischen Spaltproduktes findet nicht statt.

Die Trennung des Produktstroms erfolgt zweckmäßigerweise bei Temperaturen unter 60 °C. Die organische Phase kann dann durch destillative Trennung weiter aufgearbeitet werden.

## Beispiel

Unter Einsatz des im folgenden beschriebenen einstufigen Reaktors und einem angeschlossenen zweistufig betriebenen Spaltreaktor werden im Zweischrichtbetrieb an zweimal fünf aufeinanderfolgenden Tagen 4 500 kg β-Pinen umgesetzt. Die Bedingungen für die Ozonisierung waren wie folgt :

| | |
|---|---|
| Einsatzprodukt : | 37,5 kg/h β-Pinen (92,5 %ig) |
| | 37,5 kg/h Caprylsäure |
| | 50 kg/h Wasser |
| Luftmenge : | 670-740 $Nm^3/h$ |
| Ozoneinsatz : | 10,4-11,0 kg/h (= 3-8 % Überschuß) |
| Maximaltemperatur am Reaktorausgang : | ca. 40-45 °C |

Das Temperaturprofil im Reaktor stellt sich selbstregelnd ein, und zwar ansteigend vom Eintritt bis Austritt. Der Reaktor arbeitete praktisch ohne Überwachung oder Regelung.

Als Reaktor wurde eine mit einer geometrisch definierten Streckmetall-Füllkörperpackung ausgerüstete Glaskolonne, NW 150, 2 800 mm Packungshöhe eingesetzt. Die Packung bietet eine Oberfläche von 250 $m^2/m^3$ zur Erzeugung eines dünnen Flüssigkeitsfilms, gleichzeitig zwingt sie dem Gas eine bypass-freie hochturbulente Zick-Zack-Strömung auf.

Der Ozonumsetzungsgrad im Einzelreaktor beträgt etwa 95 bis 98 %. Der Druckverlust macht etwa 0,1 bis 0,15 bar aus.

Die anschließende zweistufige hydrolytische Spaltung wurde mit einem Dampfdurchsatz von ca. 40 kg/h gefahren. Die Spaltkolonne war beheizt, Manteltemperatur ca. 98 °C. Produkttemperatur in der Stufe I = 103 °C, in Stuffe II = 101 °C. Der Dampf wurde nur teilweise im Spaltreaktor kondensiert.

Überschüssiger Dampf, zusammen mit wasserdampfflüchtigen Reaktionsprodukten wurde im Abgaskühler total kondensiert. Von dem anfallenden zweiphasigen Kondensat wurde die wäßrige Phase (sie enthält Ameisensäure) verworfen. Die organische Phase fiel in einer Menge von ca. 1,5 kg/h an. Die mittlere Verweilzeit des Reaktionsproduktes im Spaltreaktor betrug ca. 1 Stunde.

Die Abscheidung der bei der Ozonisierungsreaktion gebildete Nebel wird in der Abgasleitung nach Verlassen des Ozonisierungsreaktors vorgenommen. Hier waren Metallfaser-Tiefenfilterplatten installiert. Diese Metallfaserplatten sind in einer senkrechten, von oben nach unten durchströmten Abgas-Rohrleitungsstrecke montiert und werden von oben mit ca. 20 bis 100 l/h Wasser besprüht. Auf diese Weise bildet sich eine ungefährliche Öl/Wasser-Emulsion, die mit dem Abgas in den nachgeschalteten Natriumsulfitwäscher zur Vernichtung des Restozons geleitet und dort durch Reduktion unschädlich gemacht wurde.

Die Verfahrensausbeuten sind wie folgt:

Tagesanalyse

| Versuchstag | Nopinongehalt im Spaltprodukt (org. Phase) % | Nopinongehalt im Kondensat (org. Phase) % | Ozongehalt im Spaltprodukt |
|---|---|---|---|
| 1 | 24,1 | - | 0,92 |
| 2 | 23,5 | 58,4 | 0,60 |
| 3 | 24,4 | 60,0 | 0,70 |
| 4 | 22,9 | 60,4 | 0,40 |
| 5 | 24,8 | - | 0,0 |
| 6 | 23,5 | - | 0,0 |
| 7 | 24,2 | 61,9 | 0,0 |
| 8 | 25,0 | 62,5 | 0,0 |
| 9 | 25,0 | 63,0 | - |
| 10 | 23,8 | 61,0 | - |
| | Ø 24,12 % | Ø 61,0 % | |

**Patentansprüche**

1. Verfahren zur kontinuierlichen Ozonolyse von β-Pinen durch Behandeln einer Emulsion von Wasser in der Lösung des olefinischen Ausgangsmaterials in einem reaktiven organischen Lösungsmittel mit einem 0,5 bis 1,5 Gew.-% Ozon enthaltenden Gasstrom bei Gleichstromführung von flüssigem und gasförmigem Reaktanten, wobei die maximale Reaktionstemperatur auf Werte von höchstens 50 °C gesteuert wird, und Aufarbeitung des Reaktionsproduktes durch hydrolytische Spaltung, dadurch gekennzeichnet, daß man unter Vermeidung der Ausbildung von Flüssigkeitsnestern im Reaktor den flüssigen Reaktanten als turbulent strömenden Film großer Oberfläche der Einwirkung des gasförmigen Reaktanten aussetzt, dabei den gasförmigen Reaktanten mit Leerohr-Fließgeschwindigkeiten von wenigstens 2 m/sec und Verweilzeiten von weniger als 2 sec im Reaktor in hochturbulenter Zick-Zack-Strömung auf bzw. über den Flüssigkeitsfilm führt und im Abgasstrom auftretende Reaktionsnebel mittels eines auf der Anströmseite mit einer Flüssigkeit beaufschlagten Tiefenfilters bricht.

2. Verfahren nach Anspruch 1, dadurch gekennzeichnet, daß man die Reaktionsnebel mittels eines mit der flüssigen Reaktionsphase beaufschlagten Tiefenfilters bricht.

3. Verfahren nach den Ansprüchen 1 und 2, dadurch gekennzeichnet, daß man auch die beim Brechen der Reaktionsnebel abgeschiedenen Reaktionsprodukte der hydrolytischen Spaltung zuführt.

4. Verfahren nach den Ansprüchen 1 bis 3, dadurch gekennzeichnet, daß man mit Leerrohr-Fließgeschwindigkeiten von wenigstens 5 m/sec arbeitet.

5. Verfahren nach den Ansprüchen 1 bis 3, dadurch gekennzeichnet, daß man mit Leerrohr-Fließgeschwindigkeiten bis zu 15 m/sec arbeitet.

6. Verfahren nach den Ansprüchen 1 bis 3, dadurch gekennzeichnet, daß man mit Leerrohr-Fließgeschwindigkeiten von 7 bis 12 m/sec arbeitet.

7. Verfahren nach den Ansprüchen 1 bis 6, dadurch gekennzeichnet, daß man mit Verweilzeiten der Gasphase im Reaktor von weniger als 1 sec arbeitet.

8. Verfahren nach den Ansprüchen 1 bis 6, dadurch gekennzeichnet, daß man mit Verweilzeiten der Gasphase im Reaktor von weniger als 0,7 sec arbeitet.

9. Verfahren nach den Ansprüchen 1 bis 6, dadurch gekennzeichnet, daß man mit Verweilzeiten der Gasphase im Reaktor von weniger als 0,5 sec arbeitet.

10. Verfahren nach den Ansprüchen 1 bis 9, dadurch gekennzeichnet, daß man mit Reaktoren arbeitet, die geometrisch regelmäßige Füllkörperpackungen einer Oberfläche von mehr als 100 $m^2/m^3$ aufweisen.

11. Verfahren nach den Ansprüchen 1 bis 9, dadurch gekennzeichnet, daß man mit Reaktoren arbeitet, die geometrisch regelmäßige Füllkörperpackungen einer Oberfläche von mehr als 180 $m^2/m^3$ aufweisen.

12. Verfahren nach den Ansprüchen 1 bis 11, dadurch gekennzeichnet, daß im Temperaturbereich von 20 bis 50 °C bei Abführung der Reaktionswärme durch in situ Verdunstung von Wasser aus dem flüssigen Reaktanten gearbeitet wird.

13. Verfahren nach den Ansprüchen 1 bis 12, dadurch gekennzeichnet, daß mit gekühltem Reaktor — hier zweckmäßigerweise als Rohrbündel ausgebildet — im Temperaturbereich von 10 bis 20 °C gearbeitet wird.

14. Verfahren nach Ansprüchen 1 bis 13, dadurch gekennzeichnet, daß die Reaktionspartner in äquivalenten Mengenverhältnissen eingesetzt und in 1-stufiger Umsetzung zu mehr als 97 % zur Reaktion gebracht werden.

15. Verfahren nach Anspruch 14, dadurch gekennzeichnet, daß die Reaktionspartner zu über 98 % zur Reaktion gebracht werden.

16. Verfahren nach den Ansprüchen 1 bis 15, dadurch gekennzeichnet, daß mit einem Ozon enthaltenden Luftstrom bei Ozonkonzentration von 7 bis 15 $g/Nm^3$ gearbeitet wird.

17. Verfahren nach den Ansprüchen 1 bis 16, dadurch gekennzeichnet, daß als reaktive Lösungsmittel bei Verfahrenstemperatur flüssige Alkohole oder entsprechende Carbonsäuren eingesetzt werden, deren Flüchtigkeit sich von der des Reaktionsprodukts zur destillativen Abtrennung hinreichend unterscheidet.

18. Verfahren nach Anspruch 17, dadurch gekennzeichnet, daß mit Caprylsäure oder Pelargonsäure als reaktiven Lösungsmitteln gearbeitet wird.

19. Verfahren nach den Ansprüchen 1 bis 18, dadurch gekennzeichnet, daß eine Wasser/Öl-Emulsion aus 1 Mol ß-Pinen, 0,5 bis 5 Mol reaktivem Lösungsmittel und 10 bis 50 Mol Wasser eingesetzt wird.

20. Verfahren nach den Ansprüchen 1 bis 20, dadurch gekennzeichnet, daß ein Anteil des nopinonhaltigen Produkts aus der Stufe der hydrolytischen Spaltung in die Ozonisierung zurückgeführt wird.

21. Verfahren nach Anspruch 20, dadurch gekennzeichnet, daß bis zu 50 % des Nopinon enthaltenden Spaltproduktes der Rückführung unterworfen werden.

**Claims**

1. A process for the continuous ozonolysis of β-pinene by treating an emulsion of water in a solution of the olefinic starting material in a reactive organic solvent with a gas stream containing from 0.5 to 1.5 % by weight of ozone, the liquid and gaseous reactants flowing in parallel current and the maximum reaction temperature being regulated to values of at most 50 °C, and working up the reaction product by hydrolytic cleavage, characterized in that, whilst avoiding the formation of pockets of liquid in the reactor, the liquid reactant is exposed to the effect of the gaseous reactant in the form of a turbulently flowing film of large surface area, the gaseous reactant being passed through the reactor in a highly turbulent zig-zag flow along or over the liquid film at empty-tube flow rates of at least 2 m/sec and with residence times in the reactor of less than 2 secs. and reaction mists occuring in the waste gas stream being broken up by means of a depth filter charged with a liquid on the approach side.

2. A process as claimed in Claim 1, characterized in that the reaction mists are broken up by means of a depth filter charged with the liquid reaction phase.

3. A process as claimed in Claims 1 and 2, characterized in that the reaction products separated during breaking up of the reaction mists are also delivered to the hydrolytic cleavage stage.

4. A process as claimed in Claims 1 to 3, characterized in that empty-tube flow rates of at least 5 m/sec. are used.

5. A process as claimed in Claims 1 to 3, characterized in that empty-tube flow rates of up to 15 m/sec. are used.

6. A process as claimed in Claims 1 to 3, characterized in that empty-tube flow rates of from 7 to 12 m/sec. are used.

7. A process as claimed in Claims 1 to 6, characterized in that the residence time of the gas phase in the reactor is less than 1 second.

8. A process as claimed in Claims 1 to 6, characterized in that the residence time of the gas phase in the reactor is less than 0.7 second.

9. A process as claimed in Claims 1 to 6, characterized in that the residence time of the gas phase in the reactor is less than 0.5 second.

10. A process as claimed in Claims 1 to 9, characterized in that the reactors used have geometrically regular packings with a surface area of more than 100 $m^2/m^3$.

11. A process as claimed in Claims 1 to 9, characterized in that the reactors used have geometrically regular packings with a surface area of more than 180 $m^2/m^3$.

12. A process as claimed in Claims 1 to 11, characterized in that it is carried out at temperatures in the range from 20 to 50 °C, the heat of reaction being dissipated by the in situ evaporation of water from the liquid reactant.

13. A process as claimed in Claims 1 to 12, characterized in that it is carried out in a cooled reactor (best in the form of a nest of tubes) at temperatures in the range from 10 to 20 °C.

14. A process as claimed in Claims 1 to 13, characterized in that the reactants are used in equivalent quantitative ratios and are reacted to a conversion of more than 97 % in a one-stage reaction.

15. A process as claimed in Claim 14, characterized in that the reactants are reacted to a conversion of more than 98 %.

16. A process as claimed in Claims 1 to 15, characterized in that an ozone-containing air stream with an ozone concentration of from 7 to 15 $g/Nm^3$.

17. A process as claimed in Claims 1 to 16, characterized in that alcohols liquid at the process temperature or corresponding carboxylic acids, of which the volatility differs from that of the reaction product sufficiently for separation by distillation, are used as the reactive solvent.

18. A process as claimed in Claim 17, characterized in that caprylic acid or pelargonic acid is used as the reactive solvent.

19. A process as claimed in Claims 1 to 18, characterized in that a water-in-oil emulsion of 1 mole of β-pinene, from 0.5 to 5 moles of reactive solvent and from 10 to 50 moles of water is used.

20. A process as claimed in Claims 1 to 20, characterized in that a proportion of the nopinone-containing product is returned from the hydrolytic cleavage stage to the ozonization stage.

21. A process as claimed in Claim 20, characterized in that up to 50 % of the nopinone-containing cleavage product is returned.

## Revendications

1. Procédé pour l'ozonolyse en continu de β-pinène par traitement d'une émulsion d'eau dans la solution de la matière de départ oléfinique dans un solvant organique réactif, avec un courant de gaz contenant 0,5 à 1,5 % en poids d'ozone avec écoulement en courants parallèles des réactifs liquides et gazeux, la température réactionnelle maximale étant réglée à des valeurs de 50 °C maximum, ainsi que par traitement du produit réactionnel moyennant une dissociation hydrolytique, caractérisé en ce que, tout en évitant la formation de nids de liquide dans le réacteur, on expose les réactifs liquides sous forme d'une pellicule à écoulement turbulent de grande surface, à l'action des réactifs gazeux et, en l'occurrence, on fait passer les réactifs gazeux à des vitesses d'écoulement en tube vide d'au moins 2 m/seconde et avec des temps de séjour de moins de 2 secondes dans le réacteur en un écoulement en zigzag à haute turbulence ou sur la pellicule de liquide et on brise le brouillard réactionnel se formant dans le courant de gaz résiduaire, au moyen d'un filtre à lit profond sollicité par un liquide sur le côté d'afflux.

2. Procédé suivant la revendication 1, caractérisé en ce qu'on brise les brouillards réactionnels au moyen d'un filtre à lit profond sollicité par la phase réactionnelle liquide.

3. Procédé suivant les revendications 1 et 2, caractérisé en ce qu'on achemine également les produits réactionnels séparés lors de la rupture des brouillards réactionnels à la dissociation hydrolytique.

4. Procédé suivant les revendications 1 à 3, caractérisé en ce qu'on travaille à des vitesses d'écoulement en tube vide d'au moins 5 m/seconde.

5. Procédé suivant les revendications 1 à 3, caractérisé en ce qu'on travaille à des vitesses d'écoulement en tube vide allant jusqu'à 15 m/seconde.

6. Procédé suivant les revendications 1 à 3, caractérisé en ce qu'on travaille à des vitesses d'écoulement en tube vide de 7 à 12 m/seconde.

7. Procédé suivant les revendications 1 à 6, caractérisé en ce qu'on travaille à des temps de séjour de la phase gazeuse dans le réacteur de moins de 1 seconde.

8. Procédé suivant les revendications 1 à 6, caractérisé en ce qu'on travaille à des temps de séjour de la phase gazeuse dans le réacteur de moins de 0,7 seconde.

9. Procédé suivant les revendications 1 à 6, caractérisé en ce qu'on travaille à des temps de séjour de la phase gazeuse dans le réacteur de moins de 0,5 seconde.

10. Procédé suivant les revendications 1 à 9, caractérisé en ce qu'on travaille avec des réacteurs comportant des garnissages de corps de remplissage géométriquement réguliers d'une surface de plus de 100 $m^2/m^3$.

11. Procédé suivant les revendications 1 à 9, caractérisé en ce qu'on travaille avec des réacteurs comportant des garnissages de corps de remplissage géométriquement réguliers d'une surface de plus de 180 $m^2/m^3$.

12. Procédé suivant les revendications 1 à 11, caractérisé en ce qu'on travaille à une température se situant dans l'intervalle allant de 20 à 50 °C lors de la dissipation de la chaleur réactionnelle par évaporation in situ de l'eau hors des réactifs liquides.

13. Procédé suivant les revendications 1 à 12, caractérisé en ce qu'on travaille avec un réacteur refroidi (avantageusement réalisé ici sous forme d'un faisceau de tubes) à une température se situant dans l'intervalle allant de 10 à 20 °C.

14. Procédé suivant les revendications 1 à 13, caractérisé en ce qu'on utilise les partenaires réactionnels dans des rapports quantitatifs équivalents et on les met à réagir à plus de 97 % dans une réaction à une étape.

15. Procédé suivant la revendication 14, caractérisé en ce qu'on fait réagir les partenaires réactionnels à plus de 98 %.

16. Procédé suivant les revendications 1 à 15, caractérisé en ce qu'on travaille avec un courant d'air contenant de l'ozone à une concentration en ozone de 7 à 15 $g/Nm^3$.

17. Procédé suivant les revendications 1 à 16, caractérisé en ce que, comme solvants réactifs, on utilise des alcools liquides à la température opératoire ou des acides carboxyliques correspondants dont la volatilité se différencie suffisamment de celle du produit réactionnel en vue de la séparation par distillation.

18. Procédé suivant la revendication 17, caractérisé en ce qu'on travaille avec l'acide caprylique ou l'acide pélargonique comme solvant réactif.

19. Procédé suivant les revendications 1 à 18, caractérisé en ce qu'on utilise une émulsion eau/huile constituée de 1 mole de β-pinène, de 0,5 à 5 moles d'un solvant réactif et de 10 à 50 moles d'eau.

20. Procédé suivant les revendications 1 à 20, caractérisé en ce qu'on recycle, à l'ozonisation, une partie du produit contenant de la nopinone et provenant de l'étape de dissociation hydrolytique.

21. Procédé suivant la revendication 20, caractérisé en ce qu'on soumet jusqu'à 50 % du produit de dissociation contenant de la nopinone au recyclage.